# EUROPÄISCHE PATENTSCHRIFT

(11) **EP 0 615 743 B1**
(45) Veröffentlichungstag und Bekanntmachung des Hinweises auf die Patenterteilung: **03.11.1999**
(21) Anmeldenummer: 94100153.9
(22) Anmeldetag: 07.01.1994
(51) Int. Cl.: A61K 7/13

(54) **Haarfärbemittel**
Hair dyeing composition
Composition de teinture des cheveux

(30) Priorität: 22.01.1993 DE 4301663
(43) Veröffentlichungstag der Anmeldung: 21.09.1994
(73) Patentinhaber: GOLDWELL GmbH, D-64297 Darmstadt (DE)
(72) Erfinder: Lorenz, Heribert, D-64401 Gross-Bieberau (DE)

(56) Entgegenhaltungen:
- EP-A- 0 146 350
- WO-A-93/00066
- FR-A- 2 156 527

## Beschreibung

Die vorliegende Erfindung bezieht sich auf ein Haarfärbemittel auf Basis von Oxidationsfarbstoff-Vorprodukten, die unmittelbar vor der Anwendung mit einer Peroxid enthaltenden Zusammensetzung vermischt werden und mindestens eine Entwicklersubstanz sowie mindestens eine Kupplersubstanz enthalten.

Des weiteren betrifft die Erfindung die Verwendung eines speziellen N-Hydroxyethylaminotoluols als Entwicklersubstanz in Haarfarbstoff-Vorprodukten.

Haarfärbemittel bestehen, wie allgemein bekannt, üblicherweise aus zwei bis zur Anwendung voneinander getrennt gehaltenen Zusammensetzungen, von denen die eine Oxidationsfarbstoff-Vorprodukte, sogenannte Entwickler und Kuppler wie ggf. auch Nuanceure in Form direktziehender Farbstoffe und die andere, mit dieser Zusammensetzung vor der Applikation auf das Haar zu vermischende Zusammensetzung ein Peroxid, in der Regel Wasserstoffperoxid, enthält. Diese Farbstoffe führen zu einer permanenten Färbung des Haares.

Es sind bereits zahlreiche Substanzen als Entwicklersubstanzen vorgeschlagen worden; überwiegend handelt es sich dabei um verschiedene Derivate des 1,4-Diaminobenzols. In der Praxis werden nach wie vor überwiegend das p-Phenylendiamin selbst und das p-Toluylendiamin als Entwicklersubstanzen eingesetzt; eine gewisse Bedeutung hat auch das Tetraaminopyrimidin erfahren.

Diese Zusammensetzungen sind sowohl hinsichtlich ihrer dermatologischen Verträglichkeit, insbesondere der sensibilisierenden Wirkung, sowie auch der damit erzielbaren Färbeeigenschaft noch verbesserungsfähig.

Es wurde nunmehr überraschenderweise gefunden, daß Haarfärbemittel auf Basis eines Entwickler/Kupplersystems, die praktisch kein Sensibilisierungspotential aufweisen und verbesserte Farbeigenschaften über alle denkbaren Nuancen hin ergeben, dann erhalten werden, wenn als Entwicklersubstanz 2-(2'-Hydroxyethylamino)-5-aminotoluol bzw. dessen Salze in Kombination mit mindestens einer Kupplersubstanz ausgewählt aus der Gruppe Resorcin, 2-Methylresorcin, 4-Chlorresorcin, 2-Aminophenol, 3-Aminophenol, 4-(N-Methyl-amino)-phenol, 3-N,N-Dimethylaminophenol, 4-Amino-3-methylphenol, 5-Amino-2-methylphenol, 6-Amino-3-methylphenol, 3-Amino-2-methylamino-6-methoxypyridin, 2-Amino-3-hydroxypyridin, 2-Dimethylamino-5-aminopyridin, 2,6-Diaminopyridin, 1-Amino-3-(2'-hydroxyethylamino)benzol, 1-Amino-3-[bis(2'-hydroxyethyl)amino]benzol, 1,3-Diaminobenzol, 1,3-Diaminotoluol, α-Naphthol, 4-Aminodiphenylamin, 4,4'-Diaminodiphenylamin, 1,2-Diaminobenzol, 1,4-Diamino-2-chlorbenzol, 4,6-Dichlorresorcin, 4-Hydroxy-1,2-methylendioxybenzol, 1,5-Dihydroxynaphthalin, 4-Amino-1,2-methylendioxy-benzol, 1,7-Dihydroxynaphthalin, 2,7-Dihydroxynaphthalin, 2,4-Diamino-3-chlorphenol und/oder 1-Methoxy-2-amino-4-(2'-hydroxyethylamino)benzol eingesetzt werden, wobei die Anwesenheit von 1-Methoxy-2,4-diaminobenzol und 1-Ethoxy-2,4-diaminobenzol ausgeschlossen ist.

Darüberhinaus wurde festgestellt, daß auch das 2-(2'-Hydroxyethylamino)-5-aminotoluol der Formel 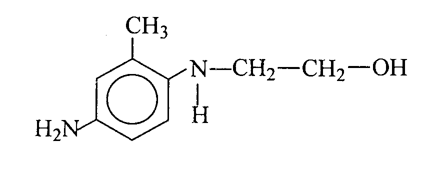 für sich eine mit sich selbst kuppelnde Wirkung aufweist und deshalb auch ohne weitere Kupplersubstanzen, gegebenenfalls im Gemisch mit an sich bekannten weiteren Entwicklersubstanzen, in Oxidationsfarbstoff-Vorprodukten eingesetzt werden kann, die nach der Vermischung mit Peroxiden zur Haarfärbung geeignet sind.

N-Hydroxyethylaminotoluole sind an sich bereits bekannt. Die Herstellung auch der erfindungsgemäß zum Einsatz gelangenden Verbindung kann in an sich bekannter Weise erfolgen, beispielsweise analog dem in Beispiel 5 der DE-A 2 240 495 beschriebenen Verfahren, ausgehend von dem entsprechenden p-Aminonitrotoluol, Umsetzung mit 2-Chlorethanol und anschließende Hydrierung.

Das erfindungsgemäß als Entwicklersubstanz in oxidativen Haarfärbemitteln eingesetzte 2-(2'-Hydroxyethyl)amino-5-toluol bzw. dessen Salze, insbesondere das Hydrochlorid oder Sulfat, kann im Gemisch mit allen möglichen Kupplersubstanzen eingesetzt werden, ausgenommen 1-Methoxy-2,4-diamonobenzol und 1-Ethoxy-2,4-diaminobenzol, mit denen die erfindungsgemäß angestrebten Effekte nicht erreicht werden und die auch aus toxikologischen Gründen völlig obsolet sind.

Insofern vermögen auch die in der EP-A 146 350 beschriebenen Zusammensetzungen keinen Hinweis in Richtung der Erfindung zu geben, da die dort beschriebenen N-(2-Hydroxyethyl)-methyl-p-phenylendiamin-Derivate ausschließlich in Kombination mit derartigen Kupplern eingesetzt werden.

Aus der EP-A 414 585 sind Haarfärbemittel bekannt, die ein p-Phenylendiamin-Derivat mit einer sekundären Aminogruppe und einem Indol-Farbstoff, vorzugsweise 5,6-Dihydroxyindol, enthalten. Die dort beschriebene Kombination soll die Haarfärbung durch die Einwirkung von Luftsauerstoff, ohne Gegenwart eines Oxidationsmittels, ermöglichen und steht daher mit der vorliegenden Erfindung hinsichtlich Aufgabenstellung und Lösung in keinem näheren Zusammenhang.

Das 2-(2'-Hydroxyethylamino)-5-aminotoluol wird in den erfindungsgemäßen Haarfärbemitteln vorzugsweise als alleinige Entwicklersubstanz in einer Menge von etwa 0,05 bis etwa 5 Gew.-%, vorzugsweise 0,1 bis 3, insbesondere 0,25 bis 1,5 Gew.-%, berechnet auf die Gesamtzusammensetzung des peroxidfreien Mittels und, falls es in Form eines Salzes vorliegt, auf die freie Base eingesetzt, jedoch ist in speziellen Fällen, falls die Erzielung besonderer Nuancen gewünscht wird, die Mitverwendung weiterer Entwicklersubstanzen in untergeordneten Mengen möglich, beispielsweise von p-Phenylendiamin oder p-Toluylendiamin, 4-Aminophenol, Tetraaminopyrimidinen oder Triaminohydroxypyrimidinen.

Die gesamten Kupplersubstanzen werden in einem molaren Verhältnis von Entwicklersubstanz zu Kupplersubstanz zwischen etwa 1:1 und etwa 2,5:1 verwendet.

Es können selbstverständlich auch, zur Erzielung besonderer Farbtöne, Gemische aus verschiedenen Kupplersubstanzen eingesetzt werden, insbesondere solche aus Resorcin oder 2-Methylresorcin mit 2-Aminophenol und/oder 3-Aminophenol, aus Resorcin oder 2-Methylresorcin und 1-Methoxy-2-amino-4-(2'-hydroxyethylamino)benzol, Gemische aus 2-Amino-3-hydroxypyridin mit 5-Amino-2-methylphenol und/oder 1-Methoxy-2-amino-4-(2'-hydroxyethylamino)benzol, oder auch 1,3-Diaminobenzol mit 1,4-Diamino-2-chlorbenzol.

Falls erwünscht, können zur Erzielung bestimmter Farbnuancen auch übliche direktziehende Farbstoffe, beispielsweise die bekannten Arianor-Farbstoffe oder auch Nitrofarbstoffe wie 2-Amino-4,6-dinitrophenol, 2-Amino-6-chlor-4-nitrophenol, 2-Amino-4-nitrophenol etc., sowie auch bekannte Pflanzenfarbstoffe, beispielsweise Henna, in geringen Mengen, d.h. zwischen 0,05 und 1 Gew.-%, enthalten sein. Ausgeschlossen ist die Mitverwendung von 2-Nitro-p-phenylendiamin und 4-Nitro-o-phenylendiamin aufgrund ihrer toxikologischen Bedenklichkeit. Die Gesamtmenge des Farbstoffgemisches im Endprodukt beträgt vorzugsweise etwa 0,2 bis etwa 6,0 Gew.-%, insbesondere etwa 0,5 bis etwa 4 Gew.-%, des Haarfärbemittels.

Zur Herstellung des erfindungsgemäßen Haarfärbemittels werden die Oxidationsfarbstoff-Vorprodukte, d.h. das Gemisch aus Entwicklersubstanz und Kupplersubstanz und ggf. anwesenden direktziehenden Farbstoffen, in einen geeigneten kosmetischen Träger eingearbeitet. Solche sind insbesondere Emulsionen, d.h. Cremes, oder Gele.

Solche Zusammensetzungen und die in ihnen enthaltenen weiteren Stoffe, insbesondere oberflächenaktive Substanzen, Stabilisatoren, Verdickungsmittel etc. sind einschlägiger Stand der Technik und in verschiedenen Monographien, beispielsweise bei K.Schrader, "Grundlagen und Rezepturen der Kosmetika", 2. Auflage (1989, Hüthig Buchverlag), S. 796 bis 815, beschrieben. Auf diese und andere Monographien wird daher ausdrücklich Bezug genommen.

Die erfindungsgemäßen Zusammensetzungen werden unmittelbar vor der Anwendung mit peroxidhaltigen Zusammensetzungen, beispielsweise mit jeweils gleichen Anteilen 6prozentiger Wasserstoffperoxid-Lösung, vermischt und auf menschliches Haar aufgebracht, wo sie nach etwa 15- bis 30-minütiger Einwirkung mit Wasser und einem üblichen Shampoo wieder ausgewaschen werden.

Die an sich bevorzugt verwendeten Wasserstoffperoxid-Zusammensetzungen können durch andere Peroxid-Zubereitungen ersetzt sein, beispielsweise Perborate, Harnstoffperoxid, Melaminperoxid, etc.; jedoch gestaltet sich die Dosierung und Handhabung solcher Zubereitungen, die bis zur Anwendung wasserfrei gehalten werden müssen, umständlicher.

Im folgenden wird die Erfindung anhand von Ausführungsbeispielen illustriert.

### Beispiele A1 - A19

In eine Grundlage aus

| | |
|---|---|
| Cetylstearylalkohol | 12,00 (Gew.-%) |
| Cocosmonoethanolamid | 2,30 |
| Stearinsäuremonoethanolamid | 2,30 |
| Propylenglykolmonostearat | 0,60 |
| Oleylalkoholethoxylat (5 EO) | 5,00 |
| Ölsäure | 2,50 |
| 1,2-Propandiol | 1,00 |
| Natriumlaurylsulfat | 0,50 |
| Komplexbildner (EDTA) | 0,50 |
| Ammoniumchlorid | 0,50 |
| Natriumsulfit | 0,50 |
| Ascorbinsäure | 0,30 |
| Eiweißhydrolysat | 1,00 |
| Parfum | 0,40 |
| Ammoniak (25%ig) | 8,00 |
| Wasser | @ 100,00 |

wurden die folgenden, mit den Nummern 1 bis 19 bezeichneten Gemische (jeweils in Gew.-%, bezogen auf die Gesamtzusammensetzung) eingebracht:

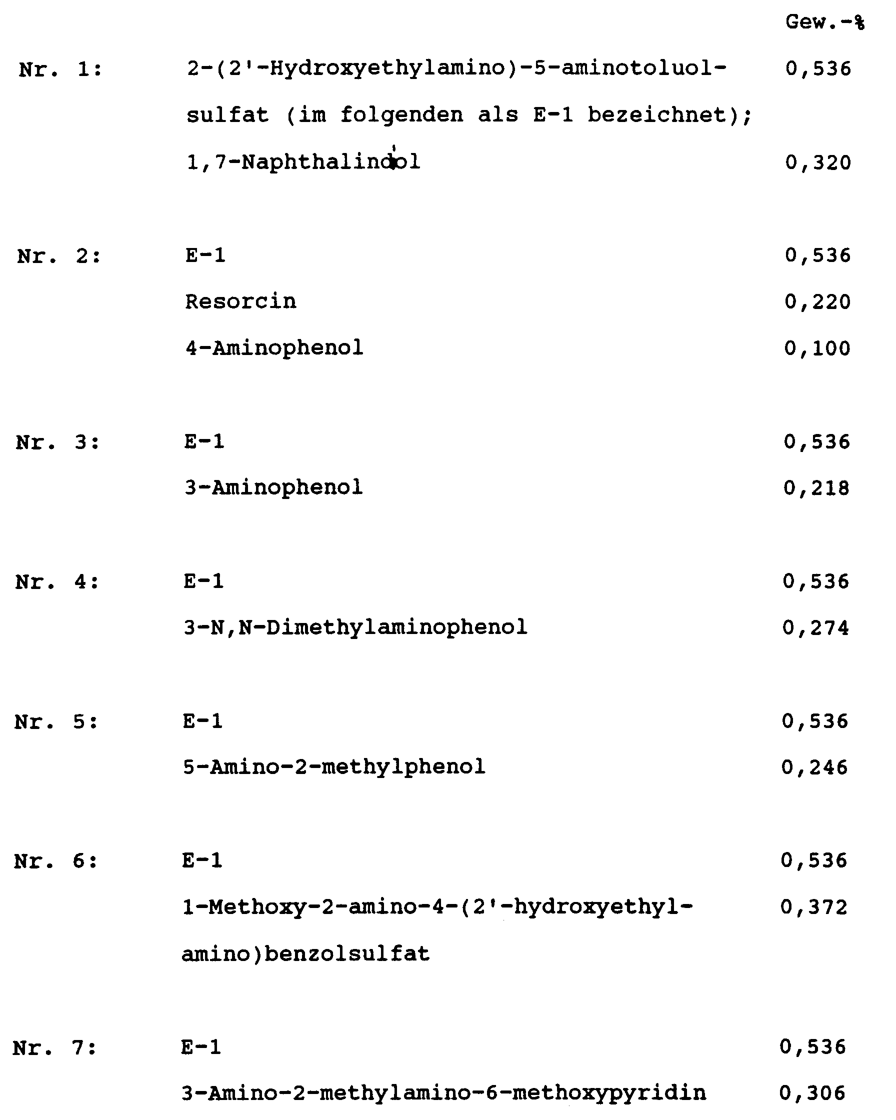

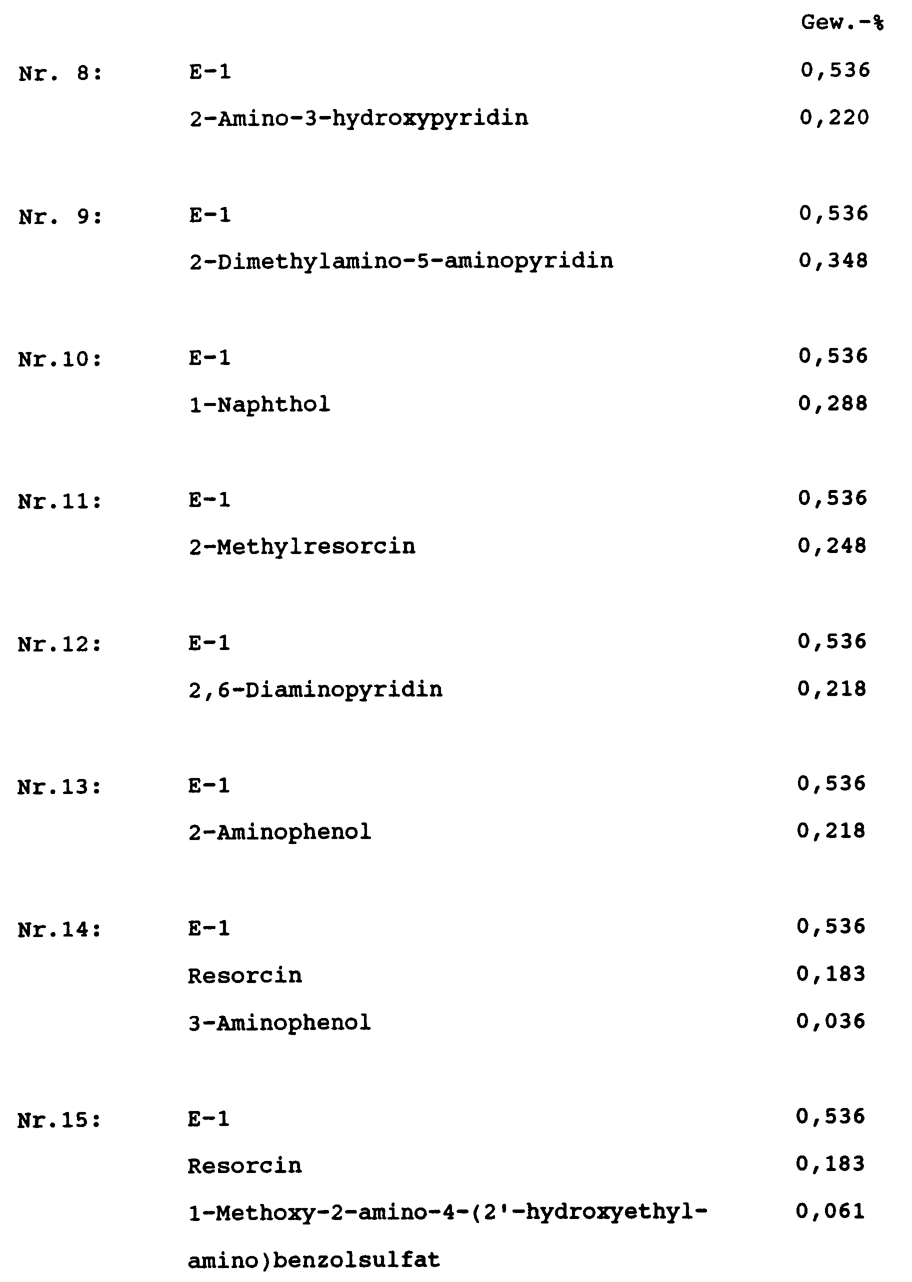

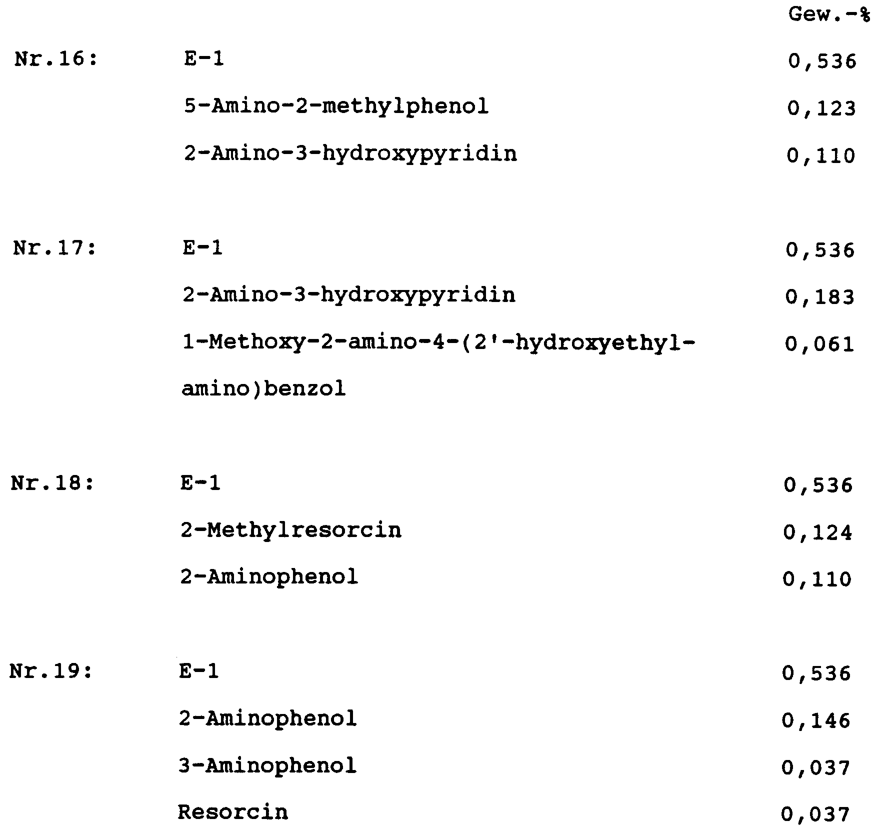

20 g dieser Zusammensetzungen wurden jeweils mit 20 ml 6prozentiger Wasserstoffperoxid-Lösung intensiv vermischt, wobei die Mischungen einen pH-Wert von etwa 9,5 aufwiesen, auf das Haar aufgebracht und nach 30-minütiger Einwirkung ausgespült, shampooniert und das Haar getrocknet. Es wurden folgende Färbungen erzielt:
- Nr. 1:: Kräftiges Blau
- Nr. 2:: Kräftiges Goldbraun
- Nr. 3:: Kräftiges Rauchgrau
- Nr. 4:: Kräftiges Olivgrün
- Nr. 5:: Kräftiges Violett
- Nr. 6:: Kräftiges Dunkelblau
- Nr. 7:: Kräftiges Blaugrau
- Nr. 8:: Kräftiges Grauviolett
- Nr. 9:: Kräftiges Graübraun
- Nr.10:: Kräftiges, leicht rotstichiges Blau
- Nr.11:: Kräftiges Graubraun
- Nr.12:: Kräftiges Türkis
- Nr.13:: Kräftiges Braunoliv
- Nr.14:: Kräftiger Aschton
- Nr.15:: Kräftiges Stahlgrau
- Nr.16:: Kräftiges Aubergine
- Nr.17:: Kräftiges Hellviolett
- Nr.18:: Kräftiges Hellgrau
- Nr.19:: Kräftiges Graubraun

Die erzielten Farbtöne beeindruckten insbesondere durch ihre ausdrucksvolle Farbkraft gegenüber konventionellen Haarfärbemitteln auf Basis der üblichen Entwicklersubstanzen.

### Beispiele B1 - B3

In eine Grundlage aus

| | |
|---|---|
| Cetylstearylalkohol | 10,00 (Gew.-%) |
| Cococfettsäuremonoethanolamid | 1,60 |
| Stearinsäuremonoethanolamid | 2,20 |
| Ölsäure | 1,00 |
| Natriumlaurylsulfat | 0,50 |
| Ammoniumchlorid | 0,25 |
| Eiweißhydrolysat | 0,60 |
| Parfum | 0,35 |
| Mangandioxid | 0,12 |
| Stabilisator, Komplexbildner | q.s. |
| Wasser | @ 100,00 |
| NaOH zur pH-Einstellung auf pH 8,5 | |

wurden die folgenden, mit den Nummern 1 bis 3 bezeichneten Entwickler/Kuppler-Gemische (jeweils in Gewichtsprozent, bezogen auf die Gesamtzusammensetzung) eingebracht:

| | | Gew.-% |
|---|---|---|
| Nr. 1: | 2-(2'-Hydroxyethylamino)-5-aminotoluolsulfat (im folgenden als E-1 bezeichnet), | 0,536 |
| | Resorcin | 0,220 |
| | 2-Aminophenol | 0,040 |
| Nr. 2: | E-1 | 0,536 |
| | 3-Aminophenol | 0,218 |
| Nr. 3: | E-1 | 0,536 |
| | Resorcin | 0,183 |
| | 3-Aminophenol | 0,036 |

20 g dieser Zusammensetzungen wurden jeweils mit 40 ml 2prozentiger Wasserstoffperoxid-Lösung intensiv vermischt, wobei ein pH-Wert von etwa 6,8 erreicht wurde, und auf das Haar aufgebracht. Nach 20-minütiger Einwirkung wurde mit Wasser gespült, shampooniert und das Haar getrocknet.

Es wurden die folgenden, ausdrucksvollen, glänzenden Farbtöne erzielt:
- Nr. 1:: Braun-Rosé
- Nr. 2:: Rauchgrau
- Nr. 3:: Grau-Rosé.

### Beispiele C1 und C2

In die in Beispiel A beschriebene Grundzusammensetzung wurden einmal
- Nr. 1:: 1,072 Gew.-% 2-(2'-Hydroxyethylamino)-5-aminotoluolsulfat
und andererseits ein Gemisch aus
- Nr. 2:: 0,536 Gew.-% 2-(2'-Hydroxyethylamino)-5-aminotoluolsulfat und
0,218 Gew.-% 4-Aminophenol
eingebracht.

Die Färbung erfolgte, wie in den Beispielen A beschrieben.

Es wurden jeweils kräftige Brauntöne erhalten.

Dies zeigt, daß auch in Abwesenheit von Kupplern durch Anwendung der erfindungsgemäßen Entwicklersubstanz allein oder im Gemisch mit einer anderen Entwicklersubstanz wie 4-Aminophenol gute Ausfärbungen erhalten werden.

Zur Erzielung der gewünschten Farbvielfalt wird jedoch in der Regel die Mitverwendung von Kupplern sinnvoll sein.

Es wurden keinerlei Hautirritationen oder Sensibilisierungen beobachtet.

## Patentansprüche

1. Haarfärbemittel auf Basis von Oxidastionsfarbstoff-Vorprodukten, die unmittelbar vor der Anwendung mit einer peroxidhaltigen Zuammensetzung vermischt werden, enthaltend mindestens eine Entwicklersubstanz und mindestens eine Kupplersubstanz, ausgenommen 1-Methoxy-2,4-diaminobenzol und 1-Ethoxy-2,4-diaminobenzol, dadurch gekennzeichnet, daß es als Entwicklersubstanz 2-(2'-Hydroxyethylamino)-5-aminotoluol und/oder dessen Salze und eine oder mehrere Kuppler-Substanzen ausgewählt aus der Gruppe Resorcin, 2-Methylresorcin, 4-Chlorresorcin, 2-Aminophenol, 3-Aminophenol, 4-(N-Methyl-amino)phenol, 3-N,N-Dimethylaminophenol, 4-Amino-3-methylphenol, 5-Amino-2-methylphenol, 6-Amino-3-methylphenol, 3-Amino-2-methylamino-6-methoxypyridin, 2-Amino-3-hydroxypyridin, 2-Dimethylamino-5-aminopyridin, 2,6-Diaminopyridin, 1-Amino-3-(2'-hydroxyethylamino)benzol, 1-Amino-3-[bis(2'-hydroxyethyl)amino]benzol, 1,3-Diaminobenzol, 1,3-Diaminotoluol, α-Naphthol, 4-Aminodiphenylamin, 4,4'-Diaminodiphenylamin, 1,2-Diaminobenzol, 1,4-Diamino-2-chlorbenzol, 4,6-Dichlorresorcin, 4-Hydroxy-1,2-methylendioxybenzol, 1,5-Dihydroxynaphthalin, 4-Amino-1,2-methylen-dioxybenzol, 1,7-Dihydroxynaphthalin, 2,7-Dihydroxynaphthalin, 2,4-Diamino-3-chlor-phenol und/oder 1-Methoxy-2-amino-4-(2'-hydroxyethylamino)benzol enthält.

2. Haarfärbemittel nach Anspruch 1, dadurch gekennzeichnet, daß es 0,05 bis 5 Gew.-%, berechnet auf die Gesamtzusammensetzung, des peroxidfreien Mittels und die freie Base, 2-(2'-Hydroxyethylamino)-5-aminotoluol enthält.

3. Haarfärbemittel nach Anspruch 1 oder 2, dadurch gekennzeichnet, daß es als Kuppler ein Gemisch aus Resorcin oder 2-Methylresorcin mit 2-Aminophenol und/oder 3-Aminophenol enthält.

4. Haarfärbemittel nach einem oder mehreren der Ansprüche 1 oder 2, dadurch gekennzeichnet, daß es als Kuppler ein Gemisch aus Resorcin oder 2-Methylresorcin mit 1-Methoxy-2-amino-4-(2'-hydroxyethylamino)benzol enthält.

5. Haarfärbemittel nach Anspruch 1 oder 2, dadurch gekennzeichnet, daß es als Kuppler ein Gemisch aus 2-Amino-3-hydroxypyridin mit 5-Amino-2-methylphenol und/oder 1-Methoxy-2-amino-4-(2'-hydroxyethylamino)benzol enthält.

6. Haarfärbemittel nach einem der vorhergehenden Ansprüche, dadurch gekennzeichnet, daß das molare Verhältnis von Entwickler und Kuppler zwischen etwa 1:1 bis 2,5:1 liegt.

7. Verwendung von 2-(2'-Hydroxyethylamino)-5-aminotoluol und/oder dessen Salzen als Entwicklersubstanz in Haarfärbemitteln auf Basis von Oxidationsfarbstoff-Vorprodukten, die keine weiteren Kupplersubstanzen enthalten und unmittelbar vor der Anwendung mit einer Peroxid enthaltenden Zusammensetzung vermischt werden.

## Claims

1. Hair dye composition based on oxidation dyestuff precursors, which are mixed with a peroxide containing composition immediately before use, comprising at least one developing agent and at least one coupling agent, excluding 1-methoxy-2,4-diaminobenzene and 1-ethoxy-2,4-diaminobenzene, characterized in that it contains 2-(2'-hydroxyethylamino)-5-aminotoluene and (or) the salts thereof as developing agent, and one are more coupling substances selected from the group of resorcinol, 2-methyl resorcinol, 4-chlororesorcinol, 2-aminophenol, 3-aminophenol, 4-(N-methyl amino)phenol, 3-N,N-dimethyl aminophenol, 4-amino-3-methyl phenol, 5-amino-2-methyl phenol, 6-amino-3-methyl phenol, 3-amino-2-methyl amino-6-methoxypyridine, 2-amino-3-hydroxy-pyridine, 2-dimethyl amino-5-aminopyridine, 2,6-diaminopyridine, 1-amino-3-(2'-hydroxy-ethyl amino)benzene, 1-amino-3-[bis(2'-hydroxyethyl)amino]benzene, 1,3-diaminobenzene, 1,3-diaminotoluene, α-naphthol, 4-aminodiphenyl amine, 4,4'-diaminodiphenyl amine, 1,2-diaminobenzene, 1,4-diamino-2-chlorbenzene, 4,6-dichlororesorcinol, 4-hydroxy-1,2-methylenedioxybenzene, 1,5-dihydroxynaphthalene, 4-amino-1,2-methylenedioxybenzene, 1,7-dihydroxynaphthaline, 2,7-dihydroxynaphthalene, 2,4-diamino-3-chlorophenol and (or) 1-methoxy-2-amino-4-(2'-hydroxyethyl amino)benzene.

2. Hair dye composition according to claim 1, characterized in that it contains 0,05 to 5 % by wt. of 2-(2'-hydroxyethyl amino)-5-aminotoluene, calculated to the total composition of the peroxide-free preparation and free base.

3. Hair dye composition according to claim 1 or 2, characterized in that it contains a mixture of resorcinol or 2-methyl resorcinol and 2-aminophenol and (or) 3-aminophenol as coupling agents.

4. Hair dye composition according to claim 1 or 2, characterized in that it contains a mixture of resorcinol or 2-methyl resorcinol and 1-methoxy-2-amino-4-(2'-hydroxyethyl amino)benzene as coupling agents.

5. Hair dye composition according to claim 1 or 2, characterized in that it contains a mixture of 2-amino-3-hydroxypyridine and 5-amino-2-methyl phenol and (or) 1-methoxy-2-amino-4-(2'-hydroxyethyl amino)benzene as coupling agents.

6. Hair dye composition according to one of the preceding claims, characterized in that the molar ratio of the developing agent to the coupling agent is from about 1:1 to 2,5:1.

7. Use of 2-(2'-hydroxyethyl amino)-5-aminotoluene and (or) the salts thereof as developing agent in hair dye compositions based on oxidation dyestuff precursors, containing no additional coupling substances, which are mixed with a peroxide compound immediately before application.

## Revendications

1. Composition de coloration des cheveux à base de précurseurs de colorants d'oxydation qui sont mélangés directement avant l'application avec une composition contenant des peroxydes, contenant au moins une substance de développement et au moins une substance de couplage, à l'exception du 1-méthoxy-2,4-diaminobenzène et du 1-éthoxy-2,4-diaminobenzène, caracté-risée en ce qu'elle contient, en tant que substance de développement, du 2- (2'-hydroxyéthylamino)-5-aminotoluène et/ou ses sels et une ou plusieurs substances de couplage choisies dans le groupe formé par la résorcine, la 2-méthylrésorcine, la 4-chlororésorcine, le 2-aminophénol, le 3-aminophénol, le 4-(N-méthylamino)phénol, le 3-N,N-diméthylaminophénol, le 4-amino-3-méthylphénol, le 5-amino-2-méthylphénol, le 6-amino-3-méthylphénol, la 3-amino-2-méthylamino-6-méthoxypyridine, la 2-amino-3-hydroxypyridine, la 2-diméthylamino-5-aminopyridine, la 2,6-diaminopyridine, le 1-amino-3-(2'-hydroxyéthylamino)benzène, le 1-amino-3-[bis(2'-hydroxyéthyl)amino]benzène, le 1,3-diaminobenzène, le 1,3-diaminotoluène, l'α-naphtol, la 4-aminodiphénylamine, la 4,4'-diaminodiphénylamine, le 1,2-diaminobenzène, le 1,4-diamino-2-chlorobenzène, la 4,6-dichlororésorcine, le 4-hydroxy-1,2-méthylènedioxybenzène, le 1,5-dihydroxynaphtalène, le 4-amino-1,2-méthylènedioxybenzène, le 1,7-dihydroxynaphtalène, le 2,7-dihydroxynaphtalène, le 2,4-diamino-3-chlorophénol et/ou le 1-méthoxy-2-amino-4-(2'-hydroxyéthylamino)-benzène.

2. Composition de coloration des cheveux selon la revendication 1, caractérisée en ce qu'elle contient 0,05 à 5 % en poids de 2-(2'-hydroxy-éthylamino)-5-aminotoluène, calculé par rapport à la composition totale exempte de peroxyde et par rapport à la base libre.

3. Composition de coloration des cheveux selon la revendication 1 ou 2, caractérisée en ce qu'elle contient, comme coupleur, un mélange de résorcine ou de 2-méthylrésorcine avec du 2-aminophénol et/ou de 3 -aminophénol.

4. Composition de coloration des cheveux selon l'une ou plusieurs des revendications 1 ou 2, caractérisée en ce qu'elle contient, comme coupleur, un mélange de résorcine ou de 2-méthylrésorcine avec du 1-méthoxy-2-amino-4-(2'-hydroxy-éthylamino) benzène.

5. Composition de coloration des cheveux selon la revendication 1 ou 2, caractérisée en ce qu'elle contient, comme coupleur, un mélange de 2-amino-3-hydroxypridine avec du 5-amino-2-méthylphénol et/ou du 1-méthoxy-2-amino-4-(2'-hydroxyéthylamino) benzène.

6. Composition de coloration des cheveux selon l'une quelconque des revendications précédentes, caractérisée en ce que le rapport molaire du développeur et du coupleur est compris entre environ 1:1 et 2,5:1.

7. Utilisation du 2-(2'-hydroxyéthylamino)-5-aminotoluène et/ou ses sels en tant que substance de développement dans des compositions de coloration des cheveux à base de précurseurs de colorants d'oxydation, qui ne contiennent aucune substance de couplage supplémentaire et qui sont mélangés directement avant l'utilisation avec une composition contenant un peroxyde.
